(19) Europäisches Patentamt
European Patent Office
Office européen des brevets

(11) EP 0 713 865 B1

(12) FASCICULE DE BREVET EUROPEEN

(45) Date de publication et mention de la délivrance du brevet:
**10.02.1999 Bulletin 1999/06**

(21) Numéro de dépôt: **95402607.6**

(22) Date de dépôt: **21.11.1995**

(51) Int Cl.[6]: **C07C 309/88**, C07C 309/46, C07C 309/48, C07C 309/52, C07C 309/49, C07D 333/20, C07D 307/52, C07D 213/38, C07D 401/06

(54) **Dérivés d'acide 2-aminobenzènesulfonique et de chlorure de 2-aminobenzènesulfonyle, leur préparation et leur utilisation comme intermédiaires de synthèse**

2-Aminobenzenesulfosäure- und 2-Aminobenzenesulfonylchloridderivate, ihre Herstellung und ihre Verwendung als Zwischenprodukte für Synthese

2-Aminobenzenesulphonic acid and 2-aminobenzenesulphonyl chloride derivatives, their preparation and their use as synthetic intermediates

(84) Etats contractants désignés:
**AT BE CH DE DK ES FR GB GR IE IT LI LU NL PT SE**

(30) Priorité: **25.11.1994 FR 9414129**

(43) Date de publication de la demande:
**29.05.1996 Bulletin 1996/22**

(73) Titulaire: **SYNTHELABO**
**92350 Le Plessis Robinson (FR)**

(72) Inventeurs:
- **Altenburger, Jean Michel**
  **F-92190 Meudon (FR)**
- **Lassalle, Gilbert**
  **F-92140 Clamart (FR)**

(74) Mandataire: **Thouret-Lemaitre, Elisabeth et al**
**SYNTHELABO,**
**Service Brevets,**
**B.P. 72**
**92352 Le Plessis-Robinson Cédex (FR)**

(56) Documents cités:
**US-A- 2 794 833**

- **HELVETICA CHIMICA ACTA, vol. 59, no. 2, 10 Mars 1976 BASEL, CH, pages 379-387, A. COURTIN: 'Bemerkungen zur Synthese von 3-Aminotoluol-5-sulfonsäure und 2-Aminotoluol-3-sulfonsäure'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 713 865 B1

Printed by Jouve, 75001 PARIS (FR)

## Description

La présente invention a pour objet des dérivés d'acide 2-aminobenzènesulfonique et de chlorure de 2-aminobenzène sulfonyle, leur préparation et leur utilisation comme intermédiaires de synthèse.

Les composés de l'invention répondent à la formule (1)

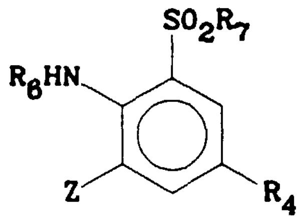

(1)

dans laquelle

$R_4$ représente soit un atome d'hydrogène, soit un atome d'halogène, soit un groupe nitro,
$R_6$ représente soit un atome d'hydrogène, soit un groupe $(C_1-C_6)$alkyle droit ou ramifié,
$R_7$ représente soit un atome de chlore, soit un groupe hydroxy et
Z représente soit un atome d'iode quand $R_7$ est un un atome de chlore, soit un groupe phényle éventuellement substitué par un ou plusieurs substituants choisis parmi les atomes d'halogènes et les groupes $(C_1-C_4)$alkyle droit ou ramifié, $(C_1-C_4)$alcoxy droit ou ramifié, trifluorométhyle, formyle, $-CH_2OR$, $-CH_2OCOR$, $-CH_2CONRR'$, $-CH_2ONCOR$, -COOR, nitro, -NHR, -NRR', -NHCOR (où R et R' sont chacun indépendamment l'un de l'autre un atome d'hydrogène ou un groupe $(C_1-C_7)$alkyle), soit un hétérocycle tels que les groupes pyridinyle, thiényle, furyle, thiazolyle et pyrimidyle, éventuellement substitués comme ci-dessus, soit un groupe cyclo$(C_5-C_8)$alkyle, quand $R_7$ est un atome de chlore ou un groupe hydroxy, sous forme libre ou sous forme de sels avec des métaux alcalins ou des amines tertiaires.

Selon l'invention les composés de formule (1) dans laquelle Z représente un atome d'iode et $R_7$ représente un atome de chlore peuvent être synthétisés à partir de l'acide sulfonique correspondant que l'on fait réagir avec du chlorure de sulfuryle en présence de triphénylphosphine ou avec du dichlorotriphénylphosphorane en présence d'une base comme la tributylamine, dans un solvant aprotique tel que par exemple le dichlorométhane.

Les composés selon l'invention pour lesquels Z représente soit un groupe phényle éventuellement substitué par un ou plusieurs substituants choisis parmi les atomes d'halogènes et les groupes $(C_1-C_4)$alkyle droit ou ramifié, $(C_1-C_4)$alcoxy droit ou ramifié, trifluorométhyle, formyle, $-CH_2OR$, $-CH_2OCOR$, $-CH_2CONRR'$, $-CH_2ONCOR$, -COOR, nitro, -NHR, -NRR', -NHCOR (où R et R' sont chacun indépendamment l'un de l'autre un atome d'hydrogène ou un groupe $(C_1-C_7)$alkyle), soit un hétérocycle choisi parmi les groupes pyridinyle, thiényle, furyle, thiazolyle et pyrimidyle, éventuellement substitués comme ci-dessus, $R_7$ un atome de chlore et $R_4$ et $R_6$ sont tels que définis précédemment, répondent à la formule (1a) (dans laquelle A est égal à Z). Ils peuvent être synthétisés selon le schéma 1.
On condense un dérivé boronique de formule (II) (dans laquelle A est tel que défini précédemment) avec un dérivé de formule (III) (dans laquelle $R_4$ représente un atome d'halogène), en présence d'un catalyseur tel que le tétrakis(triphénylphosphine)palladium (0) et d'une base comme par exemple le carbonate de sodium ou la triéthylamine, dans un solvant protique ou aprotique (tel que par exemple le 1,2-diméthoxyéthane) pour former un composé de formule (1b) qui correspond à un composé de formule (1) dans laquelle $R_6$ est un atome d'hydrogène et $R_7$ un groupe hydroxy. Ensuite,

- **voie A :** soit on fait réagir le composé de formule (1b) avec du chlorure de sulfuryle en présence de triphénylphosphine ou avec du dichlorotriphénylphosphorane, dans un solvant aprotique tel que par exemple le dichlorométhane en présence d'une base comme la tributylamine et on obtient un composé de formule (1a) (dans laquelle $R_6$ est un atome d'hydrogène et $R_4$ un atome d'halogène),
- **voie B :** soit on soumet le composé de formule (1b) à une hydrogénolyse et on obtient un composé de formule (1c) qui correspond à un composé de formule (1) dans laquelle $R_4$

Schéma 1

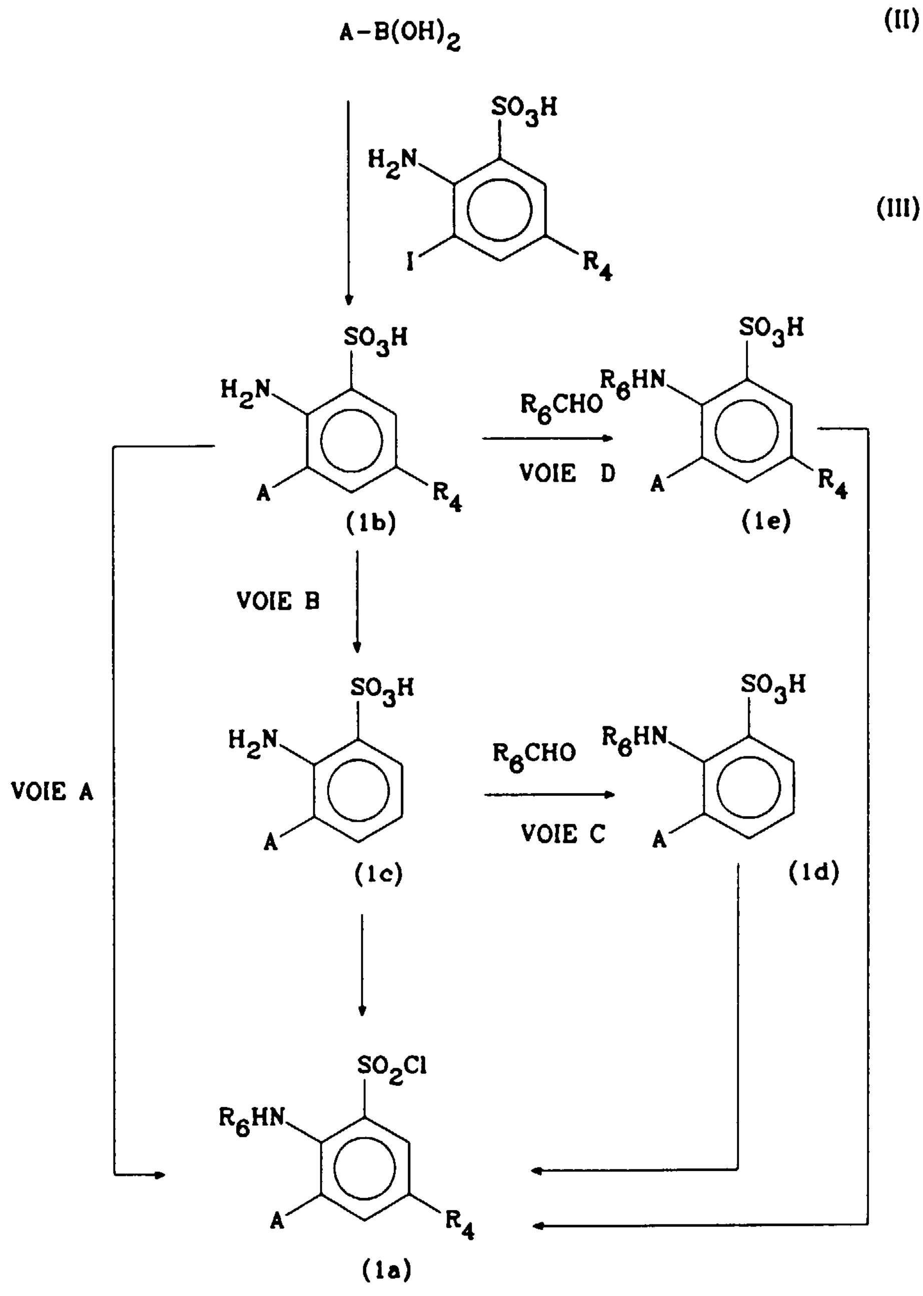


et $R_6$ représentent chacun un atome d'hydrogène et $R_7$ un groupe hydroxy, puis soit on traite le composé de formule (1c) selon la méthode décrite précédemment et on obtient un composé de formule (1a) (dans laquelle $R_4$ et $R_6$ représentent chacun un atome d'hydrogène), soit on fait réagir sur le composé de formule (1c) un aldéhyde $R_6CHO$ (où $R_6$ représente un groupe ($C_1$-$C_6$)alkyle droit ou ramifié) en milieu acide, en présence de cyanoborohydrure de sodium (**voie C**) et on obtient un composé de formule (1d) qui correspond à un composé de formule (1) dans laquelle $R_4$ représente un atome d'hydrogène $R_6$ un groupe ($C_1$-$C_6$)alkyle droit ou ramifié et $R_7$ un groupe hydroxy, ensuite, à partir du composé de formule (1d), en utilisant du dichlorotriphénylphosphorane selon la méthode décrite précédemment on prépare un composé de formule (1a) (dans laquelle $R_4$ représente un atome d'hydrogène et $R_6$ un groupe ($C_1$-$C_6$)alkyle droit ou ramifié)

- **voie D :** soit on fait réagir le composé de formule (1b) avec un aldéhyde $R_6CHO$ (où $R_6$ représente un groupe ($C_1$-$C_6$)alkyle droit ou ramifié) en milieu acide, en présence de cyanoborohydrure de sodium et on obtient un composé de formule (1e) qui correspond à un composé de formule (1) dans laquelle $R_4$ représente un atome

d'halogène, $R_6$ un groupe ($C_1$-$C_6$)alkyle droit ou ramifié et $R_7$ un groupe hydroxy, à partir duquel on prépare un composé de formule (1a) (dans laquelle $R_6$ représente un groupe ($C_1$-$C_6$)alkyle droit ou ramifié et $R_4$ un atome d'halogène), en utilisant le dichlorotriphénylphosphorane selon la méthode décrite précédemment.

Dans une variante du procédé, on peut préparer les composés de formule (1b) à partir d'un dérivé de formule A-Sn(R)$_3$ (II') où R est un groupe ($C_1$-$C_4$)alkyle que l'on fait réagir sur un composé de formule (III) sous forme de sel de triéthylamine en présence d'un catalyseur tel que le tétrakis (triphénylphosphine)palladium (0) dans un solvant protique ou aprotique tel que par exemple le diméthylformamide.

Les composés de formule (1) dans laquelle $R_4$ représente un groupe nitro sont synthétisés selon le schéma 1 (voie A) à partir de l'acide 2-amino-3-iodo-5-nitrobenzènesulfonique.

Les composés de formule (1) dans laquelle Z représente un groupe phényle ou un hétérocycle substitué par un groupe amino sont préparés par réduction du composé correspondant pour lequel Z est un groupe phényle ou un hétérocycle substitué par un groupe nitro.

Les composés de formule (1) dans laquelle Z représente un groupe phényle ou un hétérocycle substitué par un groupe -COOH sont préparés par oxydation du composé correspondant pour lequel Z est un groupe phényle ou un hétérocycle substitué par un groupe formyle.

Les composés de formule (1) dans laquelle Z représente un groupe phényle ou un hétérocycle substitué par un groupe hydroxyméthyle sont préparés par réduction du composé correspondant pour lequel Z est un groupe phényle ou un hétérocycle substitué par un groupe formyle.

Les composés de formule (1) dans laquelle Z représente un groupe phényle ou un hétérocycle substitué par un groupe électrophile peuvent être préparés à partir du composé correspondant pour lequel Z est un groupe phényle ou un hétérocycle non substitué.

Les composés selon l'invention pour lesquels Z représente un groupe cyclo($C_5$-$C_8$)alkyle et $R_4$ représente un atome d'hydrogène répondent à la formule (1f) et peuvent être synthétisés selon le schéma 2. On fait réagir un cyclo($C_5$-$C_8$)alcényle de formule (X) (dans laquelle n est égal à 1, 2, 3 ou 4) avec un composé de formule (III) (dans laquelle $R_4$ représente un atome d'halogène) en présence d'une base comme l'acétate de sodium ou de potassium, dans un solvant aprotique comme le diméthylformamide, pour former un composé de formule (XI) que l'on soumet à une hydrogénation catalytique pour obtenir un composé de formule (1g) qui correspond à un composé de formule (1) dans laquelle $R_4$ et $R_6$ représentent un atome d'hydrogène et $R_7$ un groupe hydroxy ; ensuite soit on traite le composé de formule (1g) par le dichlorotriphénylphosphorane selon la méthode décrite précédemment et on obtient un composé de formule (1f) (dans laquelle $R_6$ représente un atome d'hydrogène et n est égal à 1, 2, 3 ou 4), soit on fait réagir le composé de formule (1g) avec un aldéhyde $R_6$CHO (où $R_6$ représente un groupe ($C_1$-$C_6$)alkyle droit ou ramifié) en milieu acide, en présence de cyanoborohydrure de sodium et on obtient un composé de formule (1h) qui correspond à un composé de formule (1) dans laquelle $R_4$ représente un atome d'hydrogène, $R_6$ un groupe ($C_1$-$C_6$)alkyle droit ou ramifié et $R_7$ un groupe hydroxy, à partir duquel on prépare un composé de formule (1f) (dans laquelle $R_6$ représente un groupe ($C_1$-$C_6$)alkyle droit ou ramifié et n est égal à 1, 2, 3 ou 4),

Schéma 2

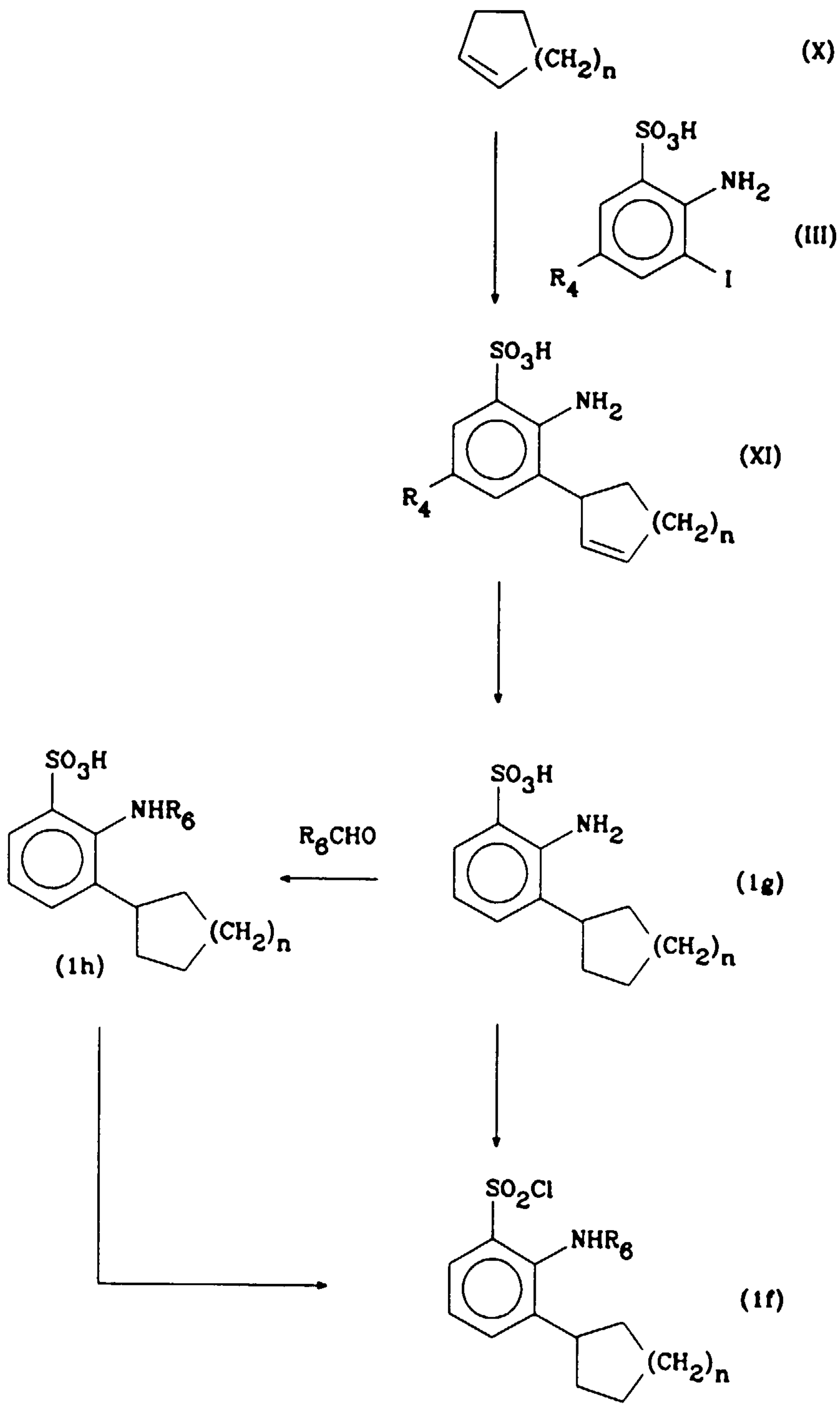


(X) (III) (XI) (1g) (1h) (1f)

en utilisant le dichlorotriphénylphosphorane selon la méthode décrite précédemment.

Les composés de départ sont disponibles dans le commerce ou décrits dans la littérature ou peuvent être préparés selon des méthodes qui y sont décrites ou qui sont connues de l'homme du métier.

Ainsi l'acide 2-amino-5-nitro-3-iodobenzènesulfonique est préparé à partir de l'acide 2-amino-5-nitro-3-benzènesulfonique selon la méthode décrite par Boyle et coll. dans J. Chem. Soc., (1919), **119**, 1505.

Les exemples 1 à 18 qui suivent illustrent la préparation de certains composés conformément à l'invention.
L'exemple A illustre l'utilisation des composés de formule (1) comme intermédiaires de synthèse.
Les microanalyses et les spectres IR et RMN confirment la structure des composés obtenus.
Les numéros des composés exemplifiés renvoient à ceux du tableau donné plus loin qui illustre les structures chimiques et les propriétés physiques de quelques composés selon l'invention.

<u>Exemple 1</u> (composé no 1)

acide 2-amino-5-bromo[1,1'-biphényle]-3-sulfonique

1.1. acide 2-amino-5-bromo-3-iodobenzènesulfonique

1.1.1. acide 2-amino-5-bromobenzènesulfonique On chauffe à 180 °C pendant 6 heures un mélange contenant 31 g (180 mmoles) de 4-bromoaniline et 9,7 ml (220 mmoles) d'acide sulfurique dans 200 ml de 1,2-dichlorobenzène. On laisse le milieu réactionnel refroidir à la température ambiante puis on le filtre. On lave le résidu par du dichlorométhane.
On obtient 45 g de produit que l'on utilise tel quel dans l'étape suivante.
Point de fusion = > 240 °C
Rendement = 97 %

1.1.2. acide 2-amino-5-bromo-3-iodobenzènesulfonique A 45 g (176 mmoles) d'acide 2-amino-5-bromobenzènesulfonique on ajoute 46 g (282 mmoles) de chlorure d'iode, 400 ml d'une solution aqueuse d'acide chlorhydrique 1 N et 400 ml de méthanol. On chauffe le mélange à 90 °C pendant 18 heures, on concentre sous pression réduite et on cristallise le résidu dans de l'éthanol.
On obtient 41 g de produit que l'on utilise tel quel dans l'étape suivante.
Point de fusion = 240 °C (décomposition)
Rendement = 62 %

1.2. acide 2-amino-5-bromo[1,1'-biphényle]-3-sulfonique A un mélange de 37,8 g (100 mmoles) d'acide 2-amino-5-bromo-3-iodobenzènesulfonique et de 32 g (300 mmoles) de carbonate de sodium dans 300 ml de 1,2-diméthoxyéthane et 150 ml d'eau, on ajoute successivement, sous atmosphère d'azote, 5,8 g (5 mmoles) de tétrakis (triphénylphosphine)palladium (0) et 19,5 g (160 mmoles) d'acide benzèneboronique. On chauffe le mélange à la température de reflux pendant 4 heures puis on concentre le milieu réactionnel sous pression réduite.
Ensuite on dissout le résidu ainsi obtenu dans un mélange contenant 300 ml de méthanol, on ajoute 300 ml d'acide chlorhydrique 0,1 N et 16 ml d'acide sulfurique à 95 %. On concentre à 100 ml sous pression réduite, on refroidit à 0 °C et on filtre. On purifie le résidu par chromatographie sur colonne phase inverse RP 18 en éluant par un mélange acétonitrile:eau (2:8).
On obtient 20 g de produit après recristallisation dans un mélange éthanol/éther.
Rendement = 60 %
Point de fusion = 197,5 °C

<u>Exemple 2</u> (composé no 2)

acide 2-amino[1,1'-biphényle]-3-sulfonique

*Voie B*

On place dans un appareil de Parr 20 g (61 mmoles) d'acide 2-amino-5-bromo[1,1'-biphényle]-3-sulfonique en présence de 3 g de palladium sur charbon à 10 % dans un mélange contenant 40 ml d'éthanol et 100 ml d'acide acétique. On chauffe le milieu réactionnel à 50 °C sous une pression de 0,35 MPa (50 psi), on le filtre sur célite et on concentre le filtrat sous pression réduite. On recristallise le résidu dans un mélange éthanol/éther.
On obtient 10 g de produit.
Rendement = 66 %
Point de fusion = 241,5 °C

Exemple 3 (composé no 3)

chlorure de 2-amino[1,1'-biphényle]-3-sulfonyle

*Voie B*

A une solution de 4,95 g (18,9 mmoles) de triphénylphosphine dans 20 ml de dichlorométhane, on ajoute goutte à goutte, à 0 °C sous atmosphère d'azote, 1,44 ml (18 mmoles) de chlorure de sulfuryle. On laisse le milieu réactionnel sous agitation pendant 10 minutes à 0 °C puis on ajoute en 5 minutes une solution contenant 2,24 g (9 mmoles) d'acide 2-amino[1,1'-biphényle]-3-sulfonique et 2,14 ml (9 mmoles) de tributylamine dans 9 ml de dichlorométhane. On porte le mélange à la température ambiante, on le laisse sous agitation à cette température pendant 3 heures et on le purifie par chromatographie sur colonne de gel de silice en éluant par un mélange pentane:dichlorométhane (8:2).
On obtient 1,9 g de produit.
Rendement = 80 %
IR, huile, $cm^{-1}$ : 3495 ; 3394 ; 3079 ; 3028 ; 1614 ; 1564 ; 1467 ; 1436 ; 1360 ; 1231 ; 1170 ; 1152 ; 1073 ; 1015 ; 839 ; 786 ; 760 ; 739 ; 704 ; 662.
RMN $^1H$, $CDCl_3$, ppm, 200 MHz : 7,8 (1H, dd, J=8 Hz, J=1,5 Hz) ; 7,55-7,3 (6H, m) ; 6,8 (1H, t, J=8 Hz) ; 5,35 (2H, s).

Exemple 4 (composé no 4)

acide 2-(propylamino)[1,1'-biphényle]-3-sulfonique

*Voie D*

A une solution contenant 5 g (0,02 mole) d'acide 2-amino [1,1'-biphényle]-3-sulfonique, 3,6 ml (0,05 mole) de propanaldéhyde et 3,28 g (0,02 mole) d'acétate de sodium dans 60 ml d'eau et 15 ml d'acide acétique, on ajoute goutte à goutte une solution de 1,9 g (0,03 mole) de cyanoborohydrure de sodium dans 15 ml d'eau. On laisse le milieu réactionnel sous agitation pendant 3 heures à la température ambiante, on le concentre et on acidifie par 80 ml d'une solution d'acide chlorhydrique 1 N et 2 ml d'une solution d'acide sulfurique à 95 %. On refroidit le mélange à 0 °C et on filtre.
On obtient 5 g de produit après recristallisation dans un mélange éthanol/éther.
Rendement = 85 %
Point de fusion = 213,5 °C

Exemple 5 (composé no 5)

chlorure de 2-(propylamino) [1,1'-biphényle]-3-sulfonyle

*Voie D*

A une solution de 8,2 g (28 mmoles) d'acide 2-(propylamino) [1,1'-biphényle]-3-sulfonique et de 6,7 ml (28 mmoles) de tributylamine dans 14 ml de dichlorométhane, on ajoute goutte à goutte à 0 °C sous atmosphère d'azote une solution de 16,95 g (42 mmoles) de dichlorotriphénylphosphorane dans 75 ml de dichlorométhane. On porte le milieu réactionnel à la température ambiante et on le maintient sous agitation à cette température pendant 6 heures.
On purifie par chromatographie sur colonne florisil® en éluant par de l'éther.
On obtient 4,35 g de produit sous forme d'une huile jaune.
Rendement = 50 %
IR, huile, $cm^{-1}$ : 3411 ; 3027 ; 2963 ; 2933 ; 2875 ; 1586 ; 1514 ; 1466 ; 1416 ; 1363 ; 1281 ; 1264 ; 1246 ; 1163 ; 1106 ; 1018 ; 798 ; 783 ; 760 ; 739 ; 702.
RMN $^1H$, $CDCl_3$, ppm, 200 MHz : 7,9 (1H, dd, J=8 Hz, J=1,7 Hz) ; 7,6-7,2 (6H, m) ; 6,9 (1H, t, J=7,6 Hz) ; 5,6 (1H, s) ; 2,6-2,5 (2H, m) ; 1,5-1,35 (2H, m) ; 0,7 (3H, t, J=7,2 Hz).

Exemple 6 (composé no 14)

2-amino-5-bromo-3'-formyl[1,1'biphényl]-3-sulfonate de sodium

On chauffe à 70 °C sous argon pendant 5 heures un mélange de 11,34 g (30 mmoles) d'acide 2-amino-5-bromo-3-iodobenzènesulfonique, 4,5 g (30 mmoles) d'acide 3-formylbenzèneboronique, 10,5 g (99 mmoles) de carbonate de sodium et 1,73 g (1,5 mmoles) de tétrakis(triphénylphosphine)palladium (0) dans 40 ml de diméthylformamide et 20 ml d'eau. On concentre ensuite le milieu réactionnel sous pression réduite et on purifie le résidu par chromatographie sur colonne RP 18 en éluant par un mélange eau/acétonitrile (8:2).
On obtient 7,4 g de produit sous forme d'une poudre blanche.
Rendement = 65 %
Point de fusion = 164-168 °C

Exemple 7 (composé no 15)

sel de *N, N,*-diéthyléthanamine de l'acide 2-amino-3'-formyl [1,1'biphényl]-3-sulfonique

On chauffe à 75 °C pendant 7 heures un mélange de 3,5 g (9,3 mmoles) de 2-amino-5-bromo-3'-formyl[1,1'biphényl]-3-sulfonate de sodium, 4,7 g (74,4 mmoles) de formiate d'ammonium, 1,1 g (11,2 mmoles) d'acétate de potassium et 0,75 g (0,65 mmoles) de tétrakis(triphénylphosphine)palladium (0) dans 20 ml de diméthylformamide. On concentre ensuite le milieu réactionnel sous pression réduite et on purifie le résidu par chromatographie sur colonne RP 18 en éluant par un mélange eau/acétonitrile (8:2). On recristallise le produit dans un mélange éthanol/éther.
On obtient 1,2 g de produit sous forme de sel de sodium sous forme d'une poudre blanche.
Rendement = 43 %
On prépare le sel *N,N,*-diéthyléthanamine selon des méthodes connues de l'homme du métier.
Point de fusion = 70-76 °C

Exemple 8 (composé no 16)

acide 2-amino-5-bromo-3'-nitro[1,1'-biphényle]-3-sulfonique

A une solution de 1,9 g (5 mmoles) d'acide 2-amino-5-bromo-3-iodobenzènesulfonique et de 1,6 g (15 mmoles) de carbonate de sodium dans 25 ml de diméthylformamide et 12,5 ml d'eau, on ajoute successivement, sous atmosphère d'azote, 0,231 g (0,2 mmole) de tétrakis(triphénylphosphine)palladium (0) et 1,42 g (8,5 mmoles) d'acide 3-nitrobenzèneboronique. On chauffe le milieu réactionnel pendant 2 heures à 70 °C et on le concentre sous pression réduite. On purifie le résidu ainsi obtenu par chromatographie sur colonne phase inverse RP 18 en éluant par un mélange acétonitrile:eau (1:9). On obtient 1,5 g de composé sous forme de sulfonate de sodium.
On libère l'acide par cristallisation de 1,5 g de sulfonate de sodium dans 80 ml de méthanol auxquels on ajoute 5 ml d'acide chlorhydrique 1 N et 0,267 µl d'acide sulfurique. On concentre, on refroidit à 0 °C, on filtre, on lave et on concentre sous pression réduite. On recristallise le produit dans un mélange éthanol/éther.
On obtient 1,3 g de produit sous forme d'une poudre jaune.
Rendement = 70 %
Point de fusion = 205 °C

Exemple 9 (composé no 17)

acide 2-amino-3'nitro[1,1'-biphényl]-3-sulfonique

On chauffe à 75 °C pendant 8 heures un mélange de 8 g (20 mmoles) de 2-amino-5-bromo-3'-nitro[1,1'biphényl]-3-sulfonate de sodium, 7,6 g (120 mmoles) de formiate d'ammonium, 2,35 g (24 mmoles) d'acétate de potassium et 1,38 g (1,2 mmoles) de tétrakis(triphénylphosphine)palladium (0) dans du diméthylformamide. On concentre ensuite le milieu réactionnel sous pression réduite et on purifie le résidu par chromatographie sur colonne RP 18 en éluant par un mélange eau/acétonitrile (8:2). On recristallise le produit dans un mélange éthanol/éther.
On obtient 2,2 g de produit sous forme de sel de sodium sous forme d'une poudre blanche.
On prépare l'acide selon la méthode décrite dans l'exemple 8.
Rendement = 35 %
Point de fusion = 228-234 °C

Exemple 10 (composé no 19)

2-amino-5-bromo-3-(thièn-2-yl)benzènesulfonate de sodium

On chauffe à 70-75 °C pendant 8,5 heures un mélange de 36 g (95,3 mmoles) d'acide 2-amino-5-bromo-3-iodobenzène sulfonique et de 12,2 g (95,3 mmoles) d'acide thièn-2-ylboronique dans 140 ml de 1,2-diméthoxyéthane en présence de 5,5 g de tétrakis(triphénylphosphine)palladium (0) et de 30,3 g de carbonate de sodium. On évapore le solvant sous vide, on refroidit à 0 °C, on lave à l'eau jusqu'à pH=7 puis on reprend le résidu dans un mélange éther/éthanol (95:5).
On obtient 21 g de produit.
Rendement = 72 %
Point de fusion = 208-214 °C

Exemple 11 (composé no 20)

2-amino-3-(thièn-2-yl)benzènesulfonate de sodium

On chauffe à 100 °C pendant 1 heure 21 g (59 mmoles) de 2-amino-5-bromo-3-(thièn-2-yl)benzènesulfonate de sodium en présence de 350 ml d'une solution à 10 % d'hydroxyde de sodium, 15 ml d'éthanol et 7,4 g de zinc. On laisse la température du milieu réactionnel revenir à la température ambiante et on ajoute 300 ml de méthanol. On filtre le mélange sur célite, on concentre le filtrat, on reprend le résidu par une solution d'hydroxyde de sodium 1 N à 0 °C puis par de l'eau à 0 °C et on sèche sous vide.
On obtient 13 g de produit.
Rendement = 75 %
Point de fusion = 224 °C

Exemple 12 (composé no 22)

sel de *N,N,* -diéthyléthanamine de l'acide 2-amino-3-(5-chlorothièn-2-yl)benzènesulfonique

A une solution de 2,57 g (9,4 mmoles) de chlorure de 2-amino-3-(thièn-2-yl)benzènesulfonyle dans 10 ml de dichlorométhane, on ajoute goutte à goutte 0,824 ml (10,3 mmoles) de chlorure de sulfuryle et on laisse le mélange sous agitation pendant 5 heures à 0 °C. Ensuite on concentre le milieu réactionnel sous pression réduite, on reprend le résidu dans 40 ml de dioxane, on ajoute à 0 °C, 20 ml d'une solution aqueuse de soude 1 N et on laisse le mélange sous agitation pendant 4 heures à 0 °C. On concentre le milieu réactionnel, on filtre, on lave le précipité et on le sèche sous pression réduite.
On obtient 2,5 g de produit sous forme de sel de sodium.
Rendement = 86 %
A une solution de 2,1 g (6,7 mmoles) dans 50 ml de méthanol on ajoute à 0°C, 7 ml d'acide chlorhydrique et 0,36 ml d'acide sulfurique concentré. On concentre le mélange sous pression réduite et on le refroidit à 0 °C. On filtre le résidu, on le lave et on le sèche sous pression réduite.
On transforme ensuite l'acide en sel de *N,N-diéthyléthanamine* selon des méthodes connues de l'homme du métier.
On obtient après cristallisation dans un mélange acétate d'éthyle/éther 1,99 g de produit.
Point de fusion = 139,4 °C

Exemple 13 (composé no 27)

sel de *N,N*,-diéthyléthanamine de l'acide 2-amino-5-bromo-3-(furan-2-yl)benzènesulfonique

On chauffe à 95 °C pendant 7 heures un mélange de 14,4 g (30 mmoles) de sel de *N,N*,-diéthyléthanamine de l'acide 2-amino-5-bromo-3-iodobenzènesulfonique, 9,92 ml (31,5 mmoles) de 2-(tributylstannyl)furanne, 0,29 g (1,5 mmoles) d'iodure de cuivre et 1,73 g (1,5 mmoles) de tétrakis(triphénylphosphine)palladium (0) dans 30 ml de diméthylformamide. On concentre ensuite le milieu réactionnel sous pression réduite et on purifie le résidu par chromatographie sur colonne de gel de silice en éluant par un mélange méthanol/dichlorométhane/triéthylamine (2:98:0,005) et on recristallise le produit dans l'acétate d'éthyle.
On obtient 6,8 g de produit sous forme de triéthylamine sous forme de cristaux orangés.
Rendement = 54 %
Point de fusion = 93-98 °C

Exemple 14 (composé no 28)

acide 2-amino-5-bromo-3-(furan-2-yl)benzènesulfonique

A une solution de 10 g (23,8 mmoles) de sel de *N, N,* -diéthyléthanamine de l'acide 2-amino-5-bromo-3-(furan-2-yl)benzènesulfonique dans 100 ml de méthanol, on ajoute à 0 °C, 24 ml d'une solution aqueuse d'acide chlorhydrique 1 N et 1,3 ml d'acide sulfurique concentré. On concentre le mélange sous pression réduite à environ 20 ml et on refroidit à 0 °C. On filtre le précipité, on le lave à l'eau et on sèche sous pression réduite. On recristallise le produit dans un mélange éthanol/éther.
On obtient 6,3 g de produit sous forme acide, sous forme d'une poudre blanche.
Rendement = 83 %
Point de fusion = 252 °C (fusion avec décomposition)

Exemple 15 (composé no 29)

sel de *N,N,*-diéthyléthanamine de l'acide 2-amino-3-(furan-2-yl)benzènesulfonique

On chauffe à 85 °C un mélange de 5,6 g (18 mmoles) d'acide 2-amino-5-bromo-3-(furan-2-yl)benzènesulfonique, 5,6 g (90 mmoles) de formiate d'ammonium, 3,5 g (36 mmoles) d'acétate de potassium et 1,04 g (0,9 mmoles) de tétrakis(triphénylphosphine)palladium (0) dans 20 ml de diméthylformamide. On laisse le mélange sous agitation pendant 1 heure à cette température puis on concentre le milieu réactionnel sous pression réduite. On purifie le résidu par chromatographie sur colonne RP 18 en éluant par un mélange eau:acétonitrile (8:2). On obtient 3 g de produit sous forme de sel de potassium sous forme d'une poudre orangée.
Rendement = 62 %
A une solution de 4,4 g (16 mmoles) de sel de potassium dans 100 ml de méthanol, on ajoute successivement à 0 °C, 20 ml d'une solution d'acide chlorhydrique 1 N et 0,85 ml d'une solution d'acide sulfurique concentré et on concentre le mélange sous pression réduite. On refroidit le mélange à 0 °C, on filtre, on lave et on sèche sous pression réduite.
On obtient 3,4 g de produit.
Rendement = 90 %
On prépare le sel de *N, N,* -diéthyléthanamine par des méthodes connues de l'homme du métier.
RMN $^1H$, $CDCl_3$, ppm, 200 MHz : 7,8 (dd, 1H, J=7,3 Hz, J=1,8 Hz) ; 7,55-7,45 (m, 2H) ; 6,75 (t, 1H, J=7 Hz) ; 6,62 (dd, 1H, J=3,6 Hz, J=0,8 Hz) ; 6,55 (dd, 1H, J=5,5 Hz, J=2 Hz) ; 5,75 (s, 2H) ; 3,15 (q, 6H, J=6,0 Hz) ; 1,35 (t, 9H, J=6,0 Hz

Exemple 16 (composé no 32)

acide 2-amino-3-cyclopentylbenzènesulfonique

7.1. acide 2-amino-5-bromo-3-cyclopent-2-èn-l-ylbenzène sulfonique
On chauffe à 80 °C sous atmosphère d'azote pendant 48 heures un mélange contenant 30 g (79,4 mmoles) d'acide 2-amino-5-bromo-3-iodobenzènesulfonique, 35 ml (397 mmoles) de cyclopentène, 19,5 g (199 mmoles) d'acétate de potassium, 6,24 g (23,8 mmoles) de triphénylphosphine et 2,67 g (11,9 mmoles) d'acétate de palladium (II) dans 160 ml de *N,N*-diméthylformamide. Ensuite on concentre le milieu réactionnel sous pression réduite et on reprend le résidu par un mélange contenant 200 ml de méthanol, 200 ml d'acide chlorhydrique 1 N et 10 ml d'une solution d'acide sulfurique à 95 %. On évapore de nouveau sous pression réduite, on filtre et on purifie le précipité ainsi obtenu par chromatographie sur colonne phase inverse RP 18 en éluant par un mélange acétonitrile: eau (3:7). On recristallise le résidu dans un mélange méthanol:pentane.
On obtient 14,9 g de produit que l'on utilise tel quel dans l'étape suivante.
Rendement = 60 %

7.2. acide 2-amino-3-cyclopentylbenzènesulfonique
On place dans un appareil de Parr 9,6 g (30,3 mmoles) d'acide 2-amino-5-bromo-3-cyclopent-2-èn-1-ylbenzènesulfonique et on ajoute 1 g de palladium sur charbon à 10 %, 80 ml de méthanol, 10 ml d'acide acétique et 50 ml d'eau. On chauffe le milieu réactionnel à 50 °C sous une pression de 0,35 MPa (50 psi) pendant 7 heures. On filtre sur célite, on concentre le filtrat sous pression réduite et on cristallise le résidu ainsi obtenu dans un mélange méthanol:pentane.
On obtient 4,9 g de produit.

Rendement = 67 %
Point de fusion = > 250 °C

Exemple 17 (composé no 33)

chlorure de 2-amino-3-cyclopentylbenzènesulfonyle

A une solution de 2,42 g (9,2 mmoles) de triphénylphosphine dans 5 ml de dichlorométhane, on ajoute goutte à goutte à 0 °C sous atmosphère d'azote, 0,70 ml (8,8 mmoles) de chlorure de sulfuryle. On laisse le milieu réactionnel sous agitation pendant 10 minutes à 0 °C puis on ajoute en 5 minutes une solution contenant 1,06 g (4,4 mmoles) d'acide 2-amino-3-cyclopentylbenzènesulfonique et 1,04 ml (4,4, mmoles) de tributylamine dans 3 ml de dichlorométhane. On porte le mélange à la température ambiante, on le laisse à cette température pendant 2 heures puis on le purifie par chromatographie sur colonne de gel de silice en éluant par un mélange dichlorométhane:pentane (1:1).
On obtient 0,8 g de produit sous forme d'une huile jaune visqueuse.
Rendement = 80 %
IR, huile, $cm^{-1}$ : 3507 ; 3408 ; 2954 ; 2869 ; 1628 ; 1565 ; 1470 ; 1357 ; 1158 ; 836 ; 739.
RMN $^1H$, $CDCl_3$, ppm, 200 MHz : 7,7 (1H, d, J=8,0 Hz) ; 7,4 (1H, d, J=8,3 Hz) ; 6,8 (1H, t, J=7,8 Hz) ; 5,4 (2H,s) ; 3,1-2,9 (1H, m) ; 2,3-1,5 (8H, m).

Exemple 18 (composé no 34)

chlorure de 2-amino-5-bromo-3-iodobenzènesulfonyle

A une solution contenant 7,6 g (20 mmoles) d'acide 2-amino-5-bromo-3-iodobenzènesulfonique et 4,8 ml (20 mmoles) de tributylamine dans 20 ml de dichlorométhane, on ajoute goutte à goutte à 0 °C sous atmosphère d'azote une solution de dichlorotriphénylphosphorane (9,7 g (37,5 mmoles) dans 45 ml de dichlorométhane). On porte le mélange à la température ambiante, on poursuit l'agitation pendant 18 heures et on le purifie par chromatographie sur colonne de gel de silice en éluant par un mélange dichlorométhane:pentane (2:8).
On obtient 6 g de produit sous forme de cristaux jaunes.
Rendement = 75 %
Point de fusion = 78 °C
RMN $^1H$, $CDCl_3$, ppm, 200 MHz : 8,05 (1H, d, J=2,65 Hz) ; 7,95 (1H, d, J=2,65 Hz) ; 5,8 (2H, s) .

Légende du tableau

*Dans la colonne "Sel" :* '$N(C_2H_5)_3$' représente un sel de *N,N*-diéthyléthanamine, 'Na' un sel de sodium et l'absence de toute mention signifie que le composé est sous forme libre.
*Dans la colonne "Point de fusion ou RMN"* : 'a' RMN dans le méthyl-$d_6$ sulfoxyde (DMSO-$d_6$); '(d)' correspond à une fusion avec décomposition

Tableau

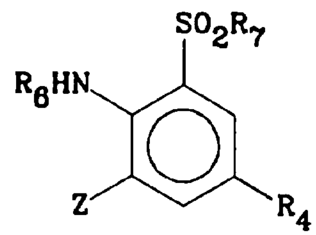

(1)

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 1 | -H | -Br | -OH | 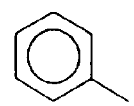 | - | 197,5 |
| 2 | -H | -H | -OH | 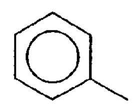 | - | 241,5 |
| 3 | -H | -H | -Cl | 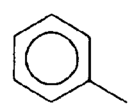 | - | 7,8 (1H, dd, J=8Hz, J=1,5Hz); 7,55-7,3 (6H, m) ; 6,8 (1H, t, J=8Hz) ; 5,35 (2H,s) |
| 4 | $-CH_2CH_2CH_3$ | -H | -OH | 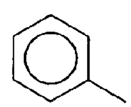 | - | 213,5 |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 5 | $-CH_2CH_2CH_3$ | -H | -Cl | 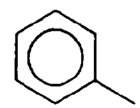 | - | 7,9 (1H, dd, J=8 Hz, J=1,7Hz) ; 7,6-7,2 (6H, m) ; 6,9 (1H, t, J=7,6Hz) ; 5,6 (1H, s) ; 2,6-2,5 (2H, m) ; 1,5-1,35 (2H, m) ; 0,7 (3H, t, J=7,2Hz) |
| 6 | -H | -H | -OH | 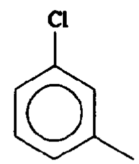 | - | 175 |
| 7 | -H | -H | -OH | 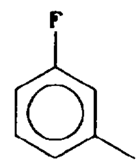 | - | 7,7 (dd, 1H, J=8,5 Hz, J=0,8Hz) ; 7,6-7,4 (m, 1H) ; 7,3-7,1 (m, 4H) ; 6,95 (t, 1H, J=8,5 Hz) [a] |
| 8 | -H | -Br | -OH | 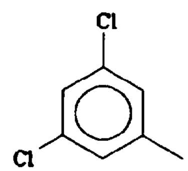 | $N(C_2H_5)_3$ | 7,75 (dd, 1H, J=8,6 Hz, J=0,7 Hz) ; 7,35 (s, 2H) ; 7,25 (s, 1H); 7,05 (dd, 1H, J=8,6 Hz, J=0,7 Hz) ; 5,05 (s, 2H) 6,7 (t, 1H, J=8,6 Hz) ; 3,15 (q, 6H, J=6 Hz) ; 1,35 (t, 9H, J=6 Hz) |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 9 | -H | -H | -OH | 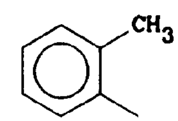 | $N(C_2H_5)_3$ | 7,7 (dd, 1H, J=8,1 Hz, J=0,6Hz) ; 7,3-7,05 (m, 4H); 6,95 (dd, 1H, J=8,1 Hz, J=0,6 Hz) ; 6,65 (t, 1H, J=8,1 Hz) ; 4,75 (s, 2H) ; 3,1 (q, 6H, J=6 Hz) ; 2,1 (s, 3H) ; 1,25 (t, 9H, J=6 Hz) |
| 10 | -H | -H | -OH | 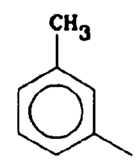 | - | 180 |
| 11 | -H | -H | -OH | 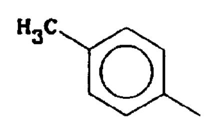 | $N(C_2H_5)_3$ | 7,75 (dd, 1H, J=8,5 Hz, J=0,7 Hz) ; 7,35-7,2 (m, 4H); 7,05 (dd, 1H, J=8,5 Hz, J=0,7 Hz) ; 6,7 (t, 1H, J=8,5 Hz) ; 5,05 (s, 2H) ; 4,6-4,4 (m, 1H) ; 3,1 (q, 6H, J=6 Hz) ; 2,3 (s, 3H) ; 1,3 (t, 9H, J=6 Hz) |
| 12 | -H | -H | -OH | 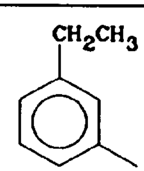 | - | 185 |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 13 | -H | -H | -OH | 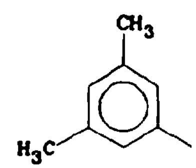 | - | >300 |
| 14 | -H | -Br | -OH | 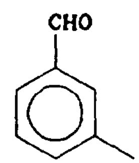 | Na | 164-168 |
| 15 | -H | -H | -OH | 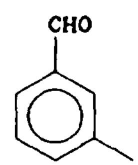 | $N(C_2H_5)_3$ | 70-76 |
| 16 | -H | -Br | -OH | 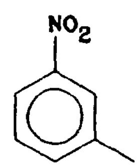 | - | 205 |
| 17 | -H | -H | -OH | 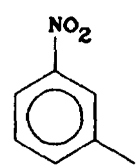 | - | 228-234 |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 18 | -H | -H | -OH | 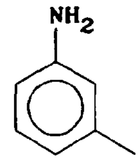 | -Na | 7,5 (dd, 1H, J=8 Hz, J=0,2 Hz) ; 7,15 (t, 1H, J=8 Hz) ; 6,9 (dd, 1H, J=8 Hz) ; 6,7-6,5 (m, 4H)[a] |
| 19 | -H | -Br | -OH | 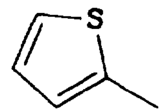 | Na | 208-214 |
| 20 | -H | -H | -OH | 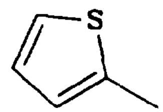 | Na | 224 |
| 21 | $-CH_2CH_2CH_3$ | -H | -OH | 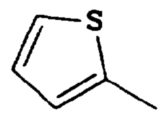 | Na | 88-92 |
| 22 | -H | -H | -OH | 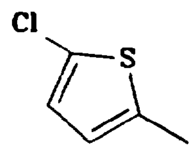 | $N(C_2H_5)_3$ | 139,4 |
| 23 | -H | -H | -OH | 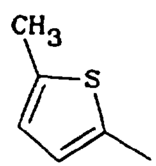 | Na | 265-270 (d) |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 24 | -H | -H | -OH | 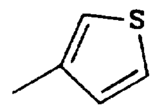 | Na | 220 |
| 25 | -H | -H | -Cl | 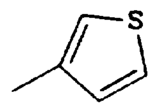 | - | 98,6 |
| 26 | -H | -Br | -OH | 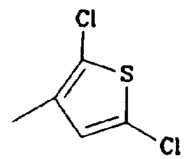 | Na | 234-238 |
| 27 | -H | -Br | -OH | 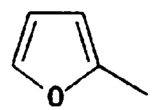 | $N(C_2H_5)_3$ | 93-98 |
| 28 | -H | -Br | -OH | 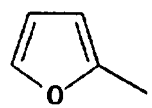 | - | 252 (d) |
| 29 | -H | -H | -OH | 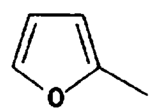 | $N(C_2H_5)_3$ | 7,8 (dd, 1H, J=7,3 Hz, J=1,8 Hz) ; 7,55-7,45 (m, 2H) ; 6,75 (t, 1H, J=7 Hz) ; 6,62 (dd, 1H, J=3,6 Hz, J=0,8 Hz) ; 6,55 (dd, 1H, J=5,5 Hz, J=2 Hz) ; 5,75 (s, 2H) ; 3,15 (q, 6H, J=6,0 Hz) ; 1,35 (t, 9H, J=6,0 Hz) |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 30 | -H | -Br | -OH | 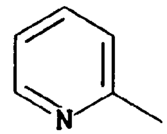 | $N(C_2H_5)_3$ | 8,7 (dd, 1H, J=6 Hz, J=0,2 Hz) ; 8-7,8 (m, 2H) ; 7,7 (d, 1H, J=0,6 Hz) ; 7,6 (d, 1H, J=0,6 Hz) ; 7,45-7,30 (m, 1H); 7,1 (s, 2H) ; 3,15 (q, 6H, J=6 Hz) ; 1,2 (t, 9H, J=6 Hz)[a] |
| 31 | -H | -H | -OH | 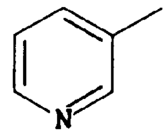 | - | 272-278 |
| 32 | -H | -H | -OH | 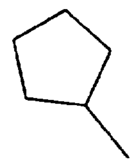 | - | > 250 |
| 33 | -H | -H | -Cl | 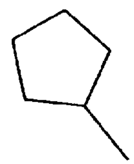 | - | 7,7 (1H, d, J=8,0Hz) ; 7,4 (1H, d, J=8,3Hz) ; 6,8 (1H, t, J=7,8Hz) ; 5,4 (2H,s) ; 3,1-2,9 (1H, m) ; 2,3-1,5 (8H, m) |
| 34 | -H | -Br | -Cl | -I | - | 78 |

| No | $R_6$ | $R_4$ | $R_7$ | Z | Sel | Point de fusion (°C) ou RMN $^1H$ $CDCl_3$, ppm, 200 MHz |
|---|---|---|---|---|---|---|
| 35 | $-CH_3$ | -H | -OH | 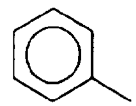 | - | 174 |
| 36 | $-CH_2CH_3$ | -H | -OH | 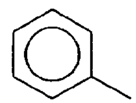 | - | 7,89 (dd, 1H) ; 7,52 (m, 6H) ; 7,40 (dd, 1H) ; 5,50 (s large , 1H échangeable) ; 2,95 (q, 2H) ; 1 (t, 3H) [a] |
| 37 | $-CH_2CH_2CH_2CH_3$ | -H | -OH | 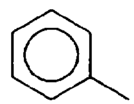 | $N(C_2H_5)_3$ | 7,8 (dd, 1H, J=8Hz, J=0,5Hz) 7,55-7,45 (m, 2H) ; 7,4-7,3 (m, 2H) ; 7,3-7,2 (m, 1H) ; 7,15 (dd, 1H, J=8Hz, J=0,5Hz) ; 6,8 (t, 1H, J=8Hz) ; 3,2 (t, 6H, J=7Hz) ; 2,55 (t, 2H, J=7Hz) ; 1,45-1,25 (m, 13H) ; 1,2-1,0 (m, 4H) ; 0,75 (t, 3H, J=6Hz) |
| 38 | $-CH_2CH(CH_3)_2$ | -H | -OH | 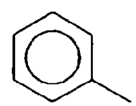 | - | 192 |

Les composés de formule (1) sont utiles entre autre comme intermédiaires dans la synthèse de composés à activité antithrombotique de formule (I)

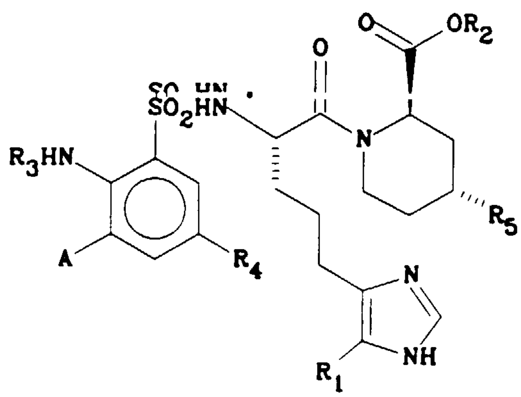

(I)

dans laquelle

$R_1$ représente soit un atome d'hydrogène, soit un groupe ($C_1$-$C_4$) alkyle,
$R_2$ représente soit un atome d'hydrogène, soit un groupe ($C_1$-$C_4$)alkyle droit ou ramifié,
$R_3$ représente soit un atome d'hydrogène, soit un groupe ($C_1$-$C_6$)alkyle droit ou ramifié,
$R_4$ représente soit un atome d'hydrogène, soit un atome d'halogène, soit un groupe nitro,
$R_5$ représente soit un atome d'hydrogène, soit un groupe ($C_1$-$C_4$)alkyle droit ou ramifié et
A représente soit un groupe phényle éventuellement substitué par un atome de fluor ou par un groupe choisi parmi les groupes ($C_1$-$C_4$)alkyle droit ou ramifié, amino et trifluorométhyle, soit un groupe cyclo($C_5$-$C_8$)alkyle, soit un hétérocycle.

L'exemple A qui suit illustre sans la limiter la synthèse des composés de formule (I) à partir des composés de l'invention.

Exemple A

chlorhydrate de [2*R*-[1(*S*), 2α, 4β]]-1- [2-[[(2-amino [1,1'-biphényl]-3-yl)sulfonyl]amino]-5-(1*H*-imidazol-4(5)-yl)-1-oxopentyl]-4-méthylpipéridine-2-carboxylate d'éthyle

A.1. [2*R*-[1(*S*), 2α, 4β]]-1-[2-[[(2-amino[1,1'-biphényl]-3-yl)sulfonyl]amino]-1-oxo-5-[1-(triphénylméthyl)-1*H*-imidazol-4-yl]pentyl]-4-méthylpipéridine-2-carboxylate d'éthyle

A une suspension de 1,8 g (3 mmoles) de chlorhydrate de [2*R*-[1(*S*), 2α, 4β]]]-1-[2-amino-1-oxo-5-[1-(triphénylméthyl)-1*H*-imidazol-4-yl]pentyl]-4-méthylpipéridine-2-carboxylate d'éthyle et de 9,2 ml (6,6 mmoles) de triéthylamine dans 12 ml de dichlorométhane, on ajoute goutte à goutte, à 0 °C sous atmosphère d'azote, 0,88 g (3,3 mmoles) de chlorure de 2-amino[l,l'-biphényle]-3-sulfonyle en solution dans 3 ml de dichlorométhane. On laisse le milieu réactionnel sous agitation à cette température pendant 6 heures puis on le concentre sous pression réduite. On purifie le résidu ainsi obtenu par chromatographie sur colonne de gel de silice en éluant par un mélange méthanol:dichlorométhane (1:99).

On obtient 2 g de produit.
Rendement = 83 %
Point de fusion = 66 °C

A.2. chlorhydrate de [2*R*-[1(*S*), 2α, 4β]]-1-[2-[[(2-amino[1,1'-biphényl]-3-yl)sulfonyl] amino]-5-(1*H*-imidazol-4 (5)-yl)-1-oxopentyl]-4-méthylpipéridine-2-carboxylate d'éthyle

Aune solution de 0,398 g (0,5 mmole) de [2*R*-[1(*S*), 2α, 4β]]-1-[2-[[(2-amino[1,1'-biphényl]-3-yl)su1fonyl]amino]-1-oxo-5-[1-(triphénylméthyl)-1H-imidazol-4-yl]pentyl]-4-méthylpipéridine-2-carboxylate d'éthyle et de 0,16 g (1,5 mmoles) d'anisole dans 15,5 ml de dichlorométhane, on ajoute goutte à goutte à 0 °C, 1,5 ml d'acide trifluoroacétique. Ensuite on laisse remonter la température du mélange à la température ambiante, on l'agite pendant 7 heures à cette température et on le concentre sous pression réduite. On reprend le résidu dans 50 ml d'acétate d'éthyle, on traite par 50 ml d'une solution saturée d'hydrogénocarbonate de sodium, on sèche sur sulfate de magnésium et on concentre sous pression réduite. On purifie le dernier résidu par chromatographie sur colonne de gel de silice en éluant par un mélange méthanol:dichlorométhane (5:95).
On obtient 0,24 g de produit sous forme de base.
Rendement = 80 %
On prépare le chlorhydrate en mettant 0,24 g (0,3 mmole) de base dans 5 ml d'une solution d'isopropanol dans l'acide chlorhydrique 0,1 N et en évaporant sous pression réduite.
On obtient 0,24 g de produit sous forme de chlorhydrate.
Point de fusion = 108 °C
$[\alpha]_D^{20}$ = 97,5 ° (c = 0,2 ; méthanol)

Les composés de formule (I) présentent une activité antithrombotique et sont décrits dans la demande de brevet français no 9414130.

## Revendications

1. Composés de formule (1)

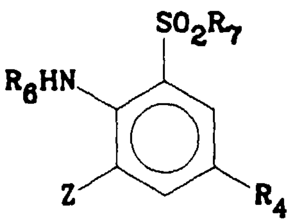

(1)

dans laquelle

$R_4$ représente soit un atome d'hydrogène, soit un atome d'halogène, soit un groupe nitro,
$R_6$ représente soit un atome d'hydrogène, soit un groupe $(C_1-C_6)$alkyle droit ou ramifié,
$R_7$ représente soit un atome de chlore, soit un groupe hydroxy et
Z représente soit un atome d'iode quand $R_7$ est un atome de chlore, soit un groupe phényle éventuellement substitué par un ou plusieurs substituants choisis parmi les atomes d'halogènes et les groupes $(C_1-C_4)$alkyle droit ou ramifié, $(C_1-C_4)$alcoxy droit ou ramifié, trifluorométhyle, formyle, $-CH_2OR$, $-CH_2OCOR$, $-CH_2CONRR'$, $-CH_2ONCOR$, -COOR, nitro, -NHR, -NRR', -NHCOR (où R et R' sont chacun indépendamment l'un de l'autre un atome d'hydrogène ou un groupe $(C_1-C_7)$alkyle), soit un hétérocycle tels que les groupes pyridinyle, thiényle, furyle, thiazolyle et pyrimidyle, éventuellement substitués comme ci-dessus, soit un groupe cyclo$(C_5-C_8)$alkyle, quand $R_7$ est un atome de chlore ou un groupe hydroxy, sous forme libre ou sous forme de sels avec des métaux alcalins ou des amines tertiaires.

2. Procédé de préparation des composés selon la revendication 1 pour lesquels Z représente un atome d'iode et $R_7$ un atome de chlore, procédé caractérisé en ce que l'on fait réagir l'acide sulfonique correspondant avec un agent de chloration en présence d'une base dans un solvant aprotique.

3. Procédé de préparation des composés de formule (1a)

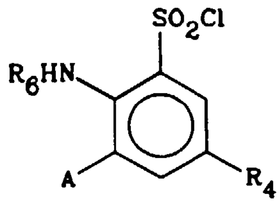

(1a)

(dans lesquels A représente soit un groupe phényle éventuellement substitué par un ou plusieurs substituants choisis parmi les atomes d'halogènes et les groupes ($C_1$-$C_4$)alkyle droit ou ramifié, ($C_1$-$C_4$)alcoxy droit ou ramifié, trifluorométhyle, formyle, $-CH_2OR$, $-CH_2OCOR$, $-CH_2CONRR'$, $-CH_2ONCOR$, -COOR, nitro, -NHR, -NRR', -NHCOR (où R et R' sont chacun indépendamment l'un de l'autre un atome d'hydrogène ou un groupe ($C_1$-$C_7$)alkyle), soit un hétérocycle choisi parmi les groupes pyridinyle, thiényle, furyle, thiazolyle et pyrimidyle, éventuellement substitués comme ci-dessus, $R_4$ et $R_6$ sont tels que définis dans la revendication 1), procédé caractérisé en ce que l'on condense un dérivé boronique de formule (II)

$$A\text{-}B\,(OH)_2 \qquad \text{(II)}$$

avec un dérivé de formule (III)

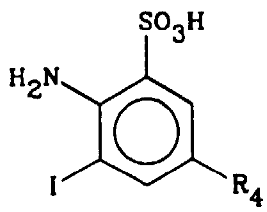

(III)

(dans laquelle $R_4$ représente un atome d'halogène)
ou un dérivé de formule

$$A\text{-}Sn\,(R_3) \qquad \text{(II')}$$

(dans laquelle R est un groupe ($C_1$-$C_4$)alkle) avec le sel de triéthylamine d'un composé de formule (III), pour former un composé de formule (1b)

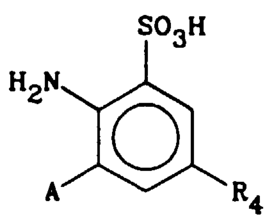

(1b)

puis,

- soit on fait réagir le composé de formule (1b) avec un agent de chloration et on obtient un composé de formule (1a) (dans laquelle $R_6$ est un atome d'hydrogène et $R_4$ un atome d'halogène),
- soit on soumet le composé de formule (1b) à une hydrogénolyse et on obtient un composé de formule (1c)

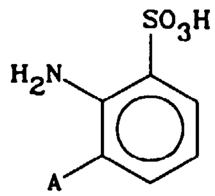

(1c)

que l'on fait réagir avec un agent de chloration et on obtient un composé de formule (1a) (dans laquelle $R_4$ et $R_6$ sont chacun un atome d'hydrogène),
ou que l'on fait réagir avec un aldéhyde $R_6CHO$ (où $R_6$ est un groupe $(C_1\text{-}C_6)$alkyle droit ou ramifié) et on obtient un composé de formule (1d)

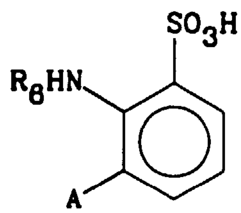

(1d)

que l'on fait réagir avec un agent de chloration et on obtient un composé de formule (1a) (dans laquelle $R_4$ est un atome d'hydrogène et $R_6$ un groupe $(C_1\text{-}C_6)$alkyle droit ou ramifié),

- soit on fait réagir le composé de formule (1b) avec un aldéhyde $R_6CHO$ (où $R_6$ est un groupe $(C_1\text{-}C_6)$alkyle droit ou ramifié) et on obtient un composé de formule (1e)

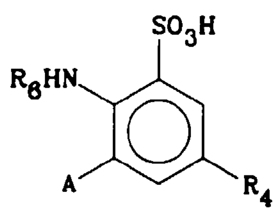

(1e)

que l'on fait réagir avec un agent de chloration et on obtient un composé de formule (1a) (dans laquelle $R_4$ est un atome d'halogène et $R_6$ un groupe $(C_1\text{-}C_6)$alkyle droit ou ramifié).

**4.** Procédé de préparation des composés de formule (1f)

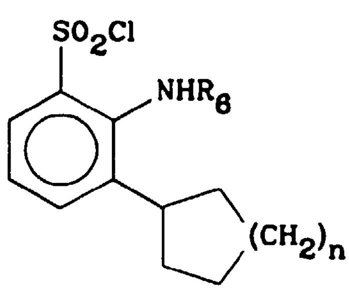

(1f)

(dans laquelle $R_6$ représente un atome d'hydrogène ou un groupe $(C_1\text{-}C_6)$alkyle droit ou ramifié et n est égal à 1, 2, 3 ou 4), procédé caractérisé en ce que l'on fait réagir un cyclo$(C_5\text{-}C_8)$alcényle de formule (X)

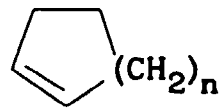

(X)

avec un composé de formule (III)

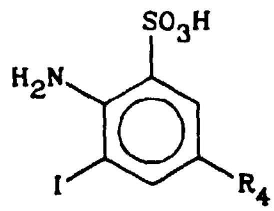

(III)

(dans laquelle $R_4$ représente un atome d'halogène) pour former un composé de formule (XI)

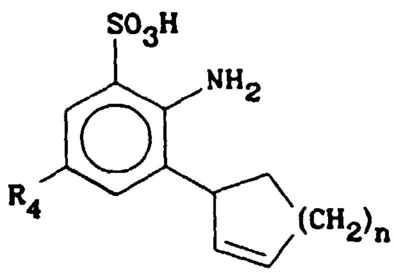

(XI)

que l'on soumet à une hydrogénation catalytique pour obtenir un composé de formule (1g)

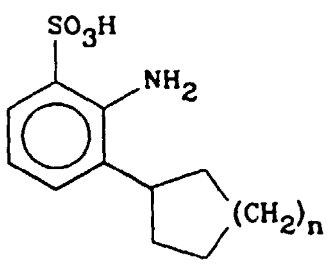

(1g)

que l'on fait réagir avec un agent de chloration et on obtient un composé de formule (1f) (dans laquelle $R_6$ représente un atome d'hydrogène et n est égal à 1, 2, 3 ou 4),
ou que l'on fait réagir avec un aldéhyde $R_6CHO$ (où $R_6$ est un groupe $(C_1$-$C_6)$alkyle droit ou ramifié) et on obtient un composé de formule (1h)

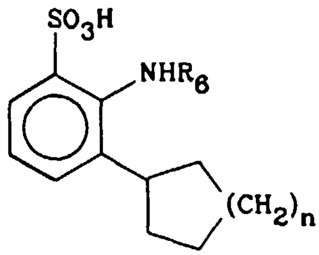

(1h)

que l'on fait réagir avec un agent de chloration et on obtient un composé de formule (1f) (dans laquelle $R_6$ est un groupe ($C_1$-$C_6$)alkyle droit ou ramifié et n est égal à 1, 2, 3 ou 4).

**5.** Procédé de préparation selon l'une quelconque des revendications 2 à 4, caractérisé en ce que l'agent de chloration est le dichlorotriphénylphosphorane.

**6.** Utilisation des composés selon la revendication 1 comme intermédiaires dans la synthèse de composés de formule (I)

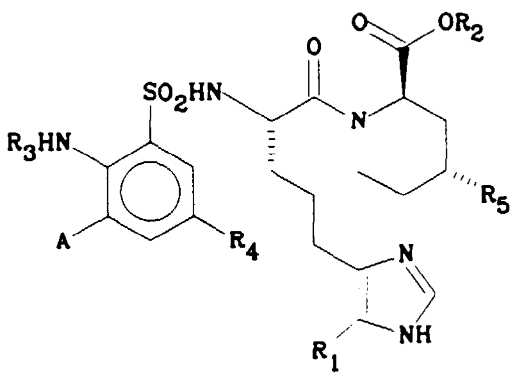

dans laquelle

$R_1$ représente soit un atome d'hydrogène, soit un groupe ($C_1$-$C_4$)alkyle,
$R_2$ représente soit un atome d'hydrogène, soit un groupe ($C_1$-$C_4$)alkyle droit ou ramifié,
$R_3$ représente soit un atome d'hydrogène, soit un groupe ($C_1$-$C_6$)alkyle droit ou ramifié,
$R_4$ représente soit un atome d'hydrogène, soit un atome d'halogène, soit un groupe nitro,
$R_5$ représente soit un atome d'hydrogène, soit un groupe ($C_1$-$C_4$)alkyle droit ou ramifié et
A représente soit un groupe phényle éventuellement substitué par un atome de fluor ou par un groupe choisi parmi les groupes ($C_1$-$C_4$)alkyle droit ou ramifié, amino et trifluorométhyle, soit un groupe cyclo($C_5$-$C_8$)alkyle, soit un hétérocycle.

**Patentansprüche**

**1.** Verbindungen der Formel (1)

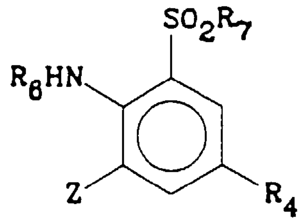

(1)

in der

$R_4$ entweder ein Wasserstoffatom, oder ein Halogenatom, oder eine Nitrogruppe,
$R_6$ entweder ein Wasserstoffatom, oder eine geradkettige oder verzweigte ($C_1$-$C_6$)-Alkylgruppe,
$R_7$ entweder ein Chloratom, oder eine Hydroxygruppe und
Z entweder ein Iodatom, wenn $R_7$ ein Chloratom darstellt, oder eine Phenylgruppe, die gegebenenfalls durch einen oder mehrere Substituenten ausgewählt aus Halogenatomen und geradkettigen oder verzweigten ($C_1$-$C_4$)-Alkylgruppen, geradkettigen oder verzweigten ($C_1$-$C_4$)-Alkoxygruppen, Trifluormethylgruppen, Formylgruppen, Gruppen der Formeln $-CH_2OR$, $-CH_2OCOR$, $-CH_2CONRR'$, $-CH_2ONCOR$, -COOR, Nitrogruppen, -NHR, -NRR', -NHCOR (worin R und R' jeweils unabhängig voneinander ein Wasserstoffatom oder eine ($C_1$-$C_7$)-Alkylgruppe darstellen) substituiert ist, oder einen Heterocyclus, wie Pyridinyl-, Thienyl-, Furyl-, Thiazolyl- und Pyrimidylgruppen, die gegebenenfalls wie oben angegeben substituiert sind, oder eine Cyclo-($C_5$-$C_8$)-alkylgruppe, wenn $R_7$ ein Chloratom oder eine Hydroxygruppe darstellt, bedeuten,
in freier Form oder in Form der Salze mit Alkalimetallen oder tertiären Aminen.

**2.** Verfahren zur Herstellung der Verbindungen nach Anspruch 1, worin Z ein Iodatom und $R_7$ ein Chloratom bedeuten, dadurch gekennzeichnet, daß man die entsprechende Sulfonsäure mit einem Chlorierungsmittel in Gegenwart einer Base in einem aprotischen Lösungsmittel umsetzt.

**3.** Verfahren zur Herstellung der Verbindungen der Formel (Ia)

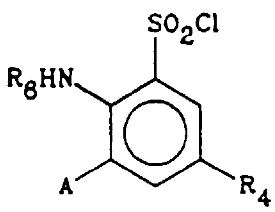

(1a)

(in der A entweder eine Phenylgruppe, die gegebenenfalls durch einen oder mehrere Substituenten ausgewählt aus Halogenatomen und geradkettigen oder verzweigten ($C_1$-$C_4$)-Alkylgruppen, geradkettigen oder verzweigten ($C_1$-$C_4$)-Alkoxygruppen, Trifluormethylgruppen, Formylgruppen, Nitrogruppen oder Gruppen der Formeln $-CH_2OR$, $-CH_2OCOR$, $-CH_2CONRR'$, $-CH_2ONCOR$, -COOR, -NHR, -NRR', -NHCOR (worin R und R' jeweils unabhängig voneinander ein Wasserstoffatom oder eine ($C_1$-$C_7$)-Alkylgruppe darstellen) substituiert ist, oder einen Heterocyclus ausgewählt aus Pyridinyl-, Thienyl-, Furyl-, Thiazolyl- und Pyrimidylgruppen, die gegebenenfalls wie oben angegeben substituiert sind, bedeutet und $R_4$ und $R_6$ die in Anspruch 1 angegebenen Bedeutungen besitzen), dadurch gekennzeichnet, daß man ein Borderivat der Formel (II)

$$A - B(OH)_2 \quad (II)$$

mit einem Derivat der Formel (III)

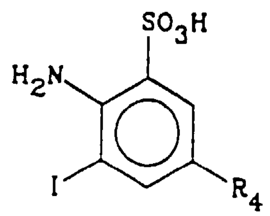

(III)

(in der $R_4$ einHalogenatom darstellt) oder einem Derivat der Formel

$$A - Sn(R_3) \qquad (II')$$

(in der R eine ($C_1$-$C_4$)-Alkylgruppe darstellt) mit dem Triethylaminsalz einer Verbindung der Formel (III) umsetzt zur Bildung einer Verbindung der Formel (1b)

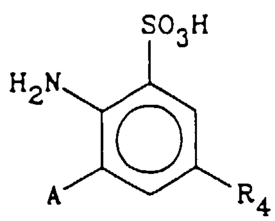

(1b)

und dann

- entweder die Verbindung der Formel (1b) mit einem Chlorierungsmittel umsetzt zur Bildung einer Verbindung der Formel (1a) (in der $R_6$ ein Wasserstoffatom und $R_4$ ein Halogenatom bedeuten),
- oder die Verbindung der Formel (1b) einer Hydrogenolyse unterwirft zur Bildung einer Verbindung der Formel (1c)

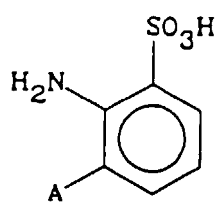

(1c)

welche man mit einem Chlorierungsmittel umsetzt zur Bildung einer Verbindung der Formel (1a) (in der $R_4$ und $R_6$ jeweils ein Wasserstoffatom bedeuten),
welche man mit einem Aldehyd $R_6CHO$ (worin $R_6$ eine geradkettige oder verzweigte ($C_1$-$C_6$)-Alkylgruppe darstellt) umsetzt zur Bildung einer Verbindung der Formel (1d)

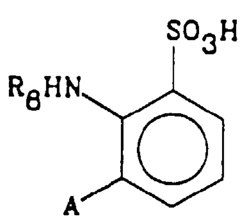

(1d)

welche man mit einem Chlorierungsmittel umsetzt zur Bildung einer Verbindung der Formel (1a) (in der $R_4$ ein Wasserstoffatom und $R_6$ eine geradkettige oder verzweigte ($C_1$-$C_6$)-Alkylgruppe bedeuten),

- oder die Verbindung der Formel (1b) mit einem Aldehyd $R_6CHO$ (worin $R_6$ eine geradkettige oder verzweigte ($C_1$-$C_6$)-Alkylgruppe darstellt) umsetzt zur Bildung einer Verbindung der Formel (1e)

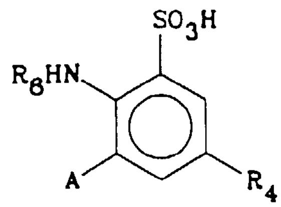

(1e)

welche man mit einem Chlorierungsmittel umsetzt zur Bildung einer Verbindung der Formel (1a) (in der $R_4$ ein Halogenatom und $R_6$ eine geradkettige oder verzweigte ($C_1$-$C_6$)-Alkylgruppe bedeuten).

**4.** Verfahren zur Herstellung der Verbindungen der Formel (1f)

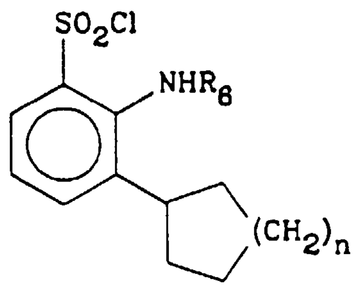

(1f)

(in der $R_6$ ein Wasserstoffatom oder eine geradkettige oder verzweigte ($C_1$-$C_6$)-Al-kylgruppe und n 1, 2, 3 oder 4 bedeuten), dadurch gekennzeichnet, daβ man ein Cyclo-($C_5$-$C_8$)-alkenyl der Formel (X)

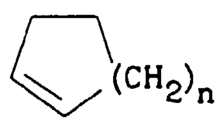

(X)

mit einer Verbindung der Formel (III)

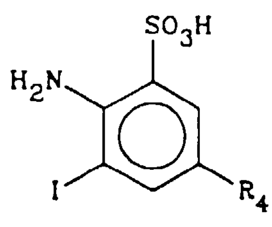

(III)

(in der $R_4$ ein Halogenatom darstellt) umsetzt
zur Bildung einer Verbindung der Formel (XI)

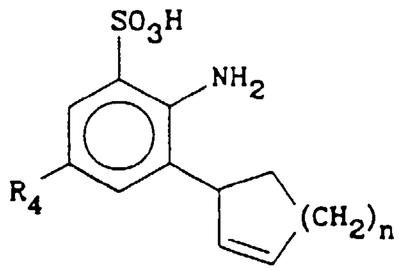

(XI)

welche man einer katalytischen Hydrierung unterwirft zur Bildung einer Verbindung der Formel (1g)

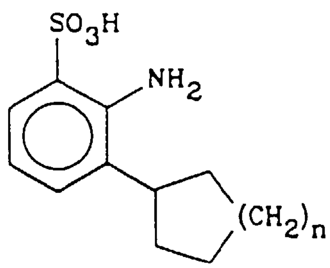

(1g)

welche man mit einem Chlorierungsmittel umsetzt zur Bildung einer Verbindung der Formel (1f) (in der $R_6$ ein Wasserstoffatom und n 1, 2, 3 oder 4 bedeuten) oder welche man mit einem Aldehyd $R_6CHO$ (worin $R_6$ eine geradkettige oder verzweigte ($C_1$-$C_6$)-Alkylgruppe darstellt) umsetzt zur Bildung einer Verbindung der Formel (1h)

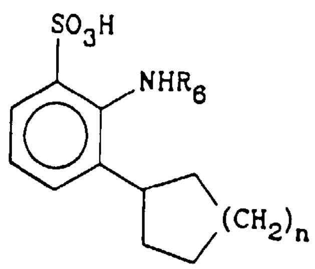

(1h)

welche man mit einem Chlorierungsmittel umsetzt zur Bildung einer Verbindung der Formel (1f) (in der $R_6$ eine geradkettige oder verzweigte ($C_1$-$C_6$)-Alkylgruppe und n 1, 2, 3 oder 4 bedeuten).

**5.** Verfahren nach irgendeinem der Ansprüche 2 bis 4, dadurch gekennzeichnet, daß man als Chlorierungsmittel Dichlortriphenylphosphoran verwendet.

**6.** Verwendung der Verbindungen nach Anspruch 1 als Zwischenprodukte bei der Synthese von Verbindungen der Formel (I)

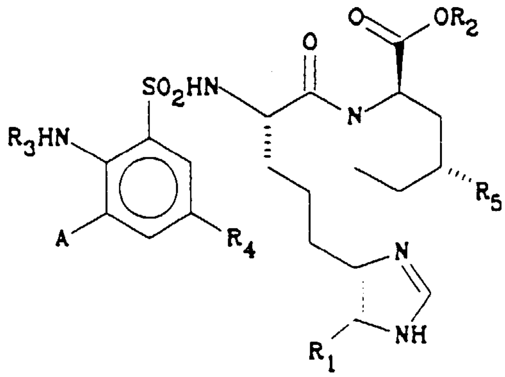

in der

$R_1$ entweder ein Wasserstoffatom oder eine $(C_1\text{-}C_4)$-Alkylgruppe,
$R_2$ entweder ein Wasserstoffatom oder eine geradkettige oder verzweigte $(C_1\text{-}C_4)$-Alkylgruppe,
$R_3$ entweder ein Wasserstoffatom oder eine geradkettige oder verzweigte $(C_1\text{-}C_6)$-Alkylgruppe,
$R_4$ entweder ein Wasserstoffatom, oder ein Halogenatom, oder eine Nitrogruppe,
$R_5$ entweder ein Wasserstoffatom, oder eine geradkettige oder verzweigte $(C_1\text{-}C_4)$-Alkylgruppe und
A entweder eine Phenylgruppe, die gegebenenfalls durch ein Fluoratom oder eine Gruppe ausgewählt aus geradkettigen oder verzweigten $(C_1\text{-}C_4)$-Alkylgruppen, Aminogruppen und Trifluormethylgruppen substituiert ist, oder eine Cyclo-$(C_5\text{-}C_8)$-alkylgruppe oder eine heterocyclische Gruppe bedeuten.

**Claims**

1. Compounds of formula (1)

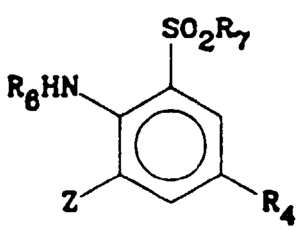

(1)

in which

$R_4$ represents either a hydrogen atom or a halogen atom or a nitro group,
$R_6$ represents either a hydrogen atom or a straight or branched $(C_1\text{-}C_6)$alkyl group,
$R_7$ represents either a chlorine atom or a hydroxyl group and
Z represents either an iodine atom when $R_7$ is a chlorine atom, or a phenyl group optionally substituted with one or more substituents chosen from halogen atoms and a straight or branched $(C_1\text{-}C_4)$alkyl group, a straight or branched $(C_1\text{-}C_4)$alkoxy group, or a trifluoromethyl, formyl, $-CH_2OR$, $-CH_2OCOR$, $-CH_2CONRR'$, $-CH_2ONCOR$, -COOR, nitro, -NHR, -NRR' or -NHCOR group (where Rand R' are each, independently of each other, a hydrogen atom or a $(C_1\text{-}C_7)$alkyl group), or a heterocycle such as pyridyl, thienyl, furyl, thiazolyl and pyrimidyl groups, optionally substituted as above, or a cyclo$(C_5\text{-}C_8)$alkyl group, when $R_7$ is a chlorine atom or a hydroxyl group, in free form or in the form of salts with alkali metals or tertiary amines.

2. Process for the preparation of the compounds according to Claim 1 for which Z represents an iodine atom and $R_7$ represents a chlorine atom, which process is characterized in that the corresponding sulphonic acid is reacted with a chlorinating agent in the presence of a base in an aprotic solvent.

**3.** Process for the preparation of the compounds of formula (1a)

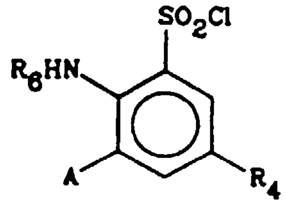

(1a)

(in which A represents either a phenyl group optionally substituted with one or more substituents chosen from halogen atoms and a straight or branched ($C_1$-$C_4$)alkyl group, a straight or branched ($C_1$-$C_4$)alkoxy group, or a trifluoromethyl, formyl, $-CH_2OR$, $-CH_2OCOR$, $-CH_2CONRR'$, $-CH_2ONCOR$, -COOR, nitro, -NHR, -NRR' or -NHCOR group (where R and R' are each, independently of each other, a hydrogen atom or a ($C_1$-$C_7$)alkyl group), or a heterocycle selected from pyridyl, thienyl, furyl, thiazolyl and pyrimidyl groups, optionally substituted as above, $R_4$ and $R_6$ are as defined in Claim 1), which process is characterized in that a boron derivative of formula (II)

$$A\text{-}B(OH)_2 \qquad (II)$$

is condensed with a derivative of formula (III)

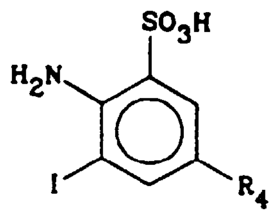

(III)

(in which $R_4$ represents a halogen atom)
or a derivative of formula

$$A\text{-}Sn(R_3) \qquad (II')$$

(in which R is a ($C_1$-$C_4$)alkyl group)
is condensed with the triethylamine salt of a compound of formula (III),
in order to form a compound of formula (1b)

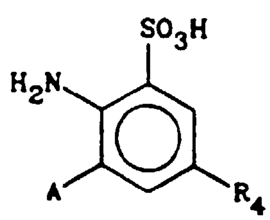

(1b)

and then,

- either the compound of formula (1b) is reacted with a chlorinating agent, and a compound of formula (1a) (in which $R_6$ is a hydrogen atom and $R_4$ is a halogen atom) is obtained,
- or the compound of formula (1b) is subjected to a hydrogenolysis, and a compound of formul1c)

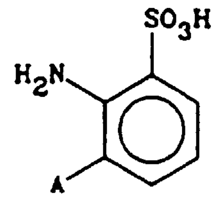

(1c)

is obtained, which is reacted with a chlorinating agent, and a compound of formula (1a) (in which $R_4$ and $R_6$ are each a hydrogen atom) is obtained,
or which is reacted with an aldehyde $R_6CHO$ (where $R_6$ is a straight or branched ($C_1$-$C_6$)alkyl group), and a compound of formula 1d)

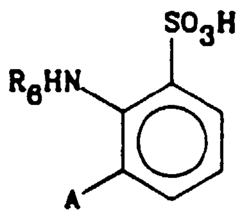

(1d)

is obtained, which is reacted with a chlorinating agent, and a compound of formula (1a) (in which $R_4$ is a hydrogen atom and $R_6$ is a straight or branched ($C_1$-$C_6$)alkyl group) is obtained,

- or the compound of formula (1b) is reacted with an aldehyde $R_6cHO$ (where $R_6$ is a straight or branched ($C_1$-$C_6$)alkyl group), and a compound of formula (1e)

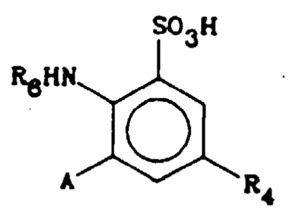

(1e)

is obtained, which is reacted with a chlorinating agent, and a compound of formula (1a) (in which $R_4$ is a halogen atom and $R_6$ is a straight or branched ($C_1$-$C_6$)alkyl group) is obtained.

**4.** Process for the preparation of the compounds of formula (1f)

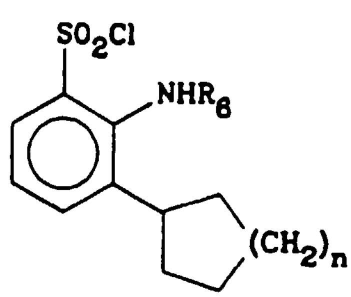

(1f)

(in which $R_6$ represents a hydrogen atom or a straight or branched ($C_1$-$C_6$)alkyl group and n is equal to 1, 2, 3 or 4), which process is characterized in that a cyclo($C_5$-$C_8$)alkenyl of formula (X)

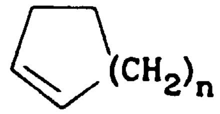

(X)

is reacted with a compound of formula (III)

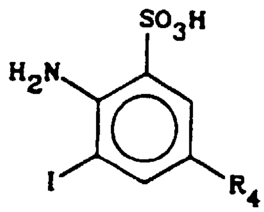

(III)

(in which $R_4$ represents a halogen atom) in order to form a compound of formula (XI)

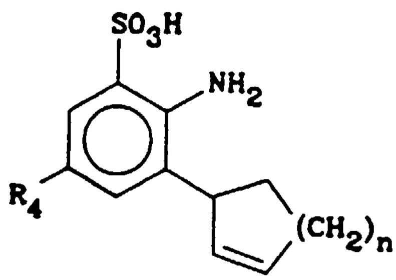

(XI)

which is subjected to catalytic hydrogenation in order to obtain a compound of formula (1g)

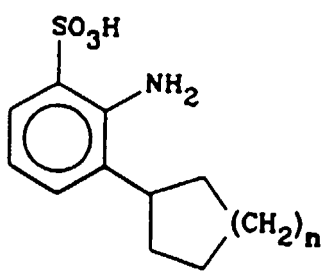

(1g)

which is reacted with a chlorinating agent, and a compound of formula (1f) (in which $R_6$ represents a hydrogen atom and n is equal to 1, 2, 3 or 4) is obtained,
or which is reacted with an aldehyde $R_6CHO$ (where $R_6$ is a straight or branched ($C_1$-$C_6$)alkyl group), and a compound of formula (1h)

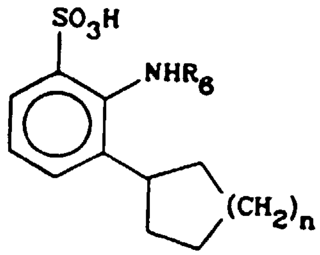

(1h)

is obtained, which is reacted with a chlorinating agent, and a compound of formula (1f) (in which $R_6$ is a straight or branched ($C_1$-$C_6$)alkyl group and n is equal to 1, 2, 3 or 4) is obtained.

**5.** Preparation process according to any one of Claims 2 to 4, characterized in that the chlorinating agent is dichlorotriphenylphosphorane.

**6.** Use of the compounds according to Claim 1 as intermediates in the synthesis of compounds of formula (I)

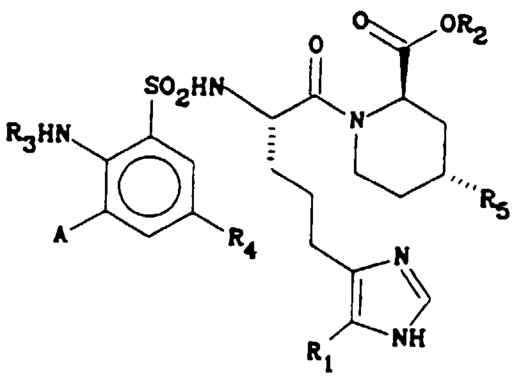

in which

$R_1$ represents either a hydrogen atom or a ($C_1$-$C_4$)alkyl group,
$R_2$ represents either a hydrogen atom or a straight or branched ($C_1$-$C_4$)alkyl group,
$R_3$ represents either a hydrogen atom or a straight or branched ($C_1$-$C_6$)alkyl group,
$R_4$ represents either a hydrogen atom or a halogen atom or a nitro group,
$R_5$ represents either a hydrogen atom or a straight or branched ($C_1$-$C_4$)alkyl group, and
A represents either a phenyl group optionally substituted with a fluorine atom or with a group chosen from a straight or branched ($C_1$-$C_4$)alkyl group, an amino group, a trifluoromethyl group, a cyclo($C_5$-$C_8$)alkyl group or a heterocycle.